# EUROPEAN PATENT SPECIFICATION

(11) **EP 2 299 939 B1**
(45) Date of publication and mention of the grant of the patent: **22.04.2015**
(21) Application number: 09735337.9
(22) Date of filing: 23.04.2009
(51) Int. Cl.: A61F 2/30, A61F 2/44

(54) **ARTIFICIAL SPINAL DISC IMPLANT**
BANDSCHEIBENPROTHESENIMPLANTAT
IMPLANT ARTIFICIEL DE DISQUE SPINAL

(30) Priority: 30.04.2008 US 112443; 24.04.2008 US 7528
(43) Date of publication of application: 30.03.2011
(73) Proprietor: Ranier Limited, Cambridge CB5 8AA (GB)
(72) Inventor: SNELL, Robert, Ashley Cambridge CB8 9ED (GB); JOHNSON, Scott, Suffolk CB8 8ET (GB); LAWSON, Jonathan, Cambridge CB5 8LF (GB)
(74) Representative: Hoffmann Eitle
(86) International application number: PCT/IB2009/005757
(87) International publication number: WO 2009/130601

(56) References cited:
- WO-A-93/01771
- WO-A-2007/133553
- FR-A- 2 893 838

## Description

### Field of Invention

The present invention generally relates to artificial spinal disc implants.

### Background of Invention

A spinal disc lies between adjacent vertebrae in the spine. The disc stabilizes the spine and assists in distributing forces between vertebral bodies. A spinal disc includes an outer annulus fibrosis which surrounds an inner nucleus pulposus. The annulus fibrosis is a concentrically laminated structure of aligned collagen fibers and fibro cartilage which provides stability to resist axial torsional and bending forces. The nucleus pulposus comprises a gelatinous material which can distribute stresses acting on the disc.

A spinal disc may be damaged due to trauma, disease or other degenerative processes that can occur over time. For example, the annulus fibrosis may weaken and/or begin to tear which can result in the protrusion of the nucleus pulposus into a region of the spine (e.g., the vertebratal foramen) that includes spinal nerves. The protruding nucleus pulposus may press against spinal nerves causing pain, numbness, tingling, diminished strength and/or a loss of motion. Another common degenerative process is the loss of fluid from the nucleus pulposus. Such fluid loss can limit the ability of the nucleus pulposus to distribute stress and may reduce its height which can lead to further instability of the spine, as well as decreasing mobility and causing pain.

To address the conditions described above, a displaced or damaged spinal disc may be surgically removed from the spine and the two adjacent vertebrae may be fused together. Though this technique may initially alleviate pain and can improve joint stability, it also can result in the loss of movement of the fused vertebral joint.

Another solution has been to replace a damaged spinal disc with an artificial spinal disc implant. However, in general, such disc implants have been limited in their ability to adequately mimic the biomechanics of a normal healthy human spinal disc. For example, such implants may not exhibit an appropriate resistance to the forces (e.g., bending, torsion, tension and compression) normally exerted on the implant throughout the day. As a result, these implants may not effectively perform the functions of a

An artificial spinal disc implant with certain biomechanical properties that better approximate those of a natural spinal disc and which may be sufficiently secured in the spine for long time periods would be desirable.

FR 2 893 838 A1 relates to an intervertebral disc prosthesis in which osseous entering means, in particular a winglet, are located closer to the posterior side.

### Summary of Invention

The present invention relates to an artificial spinal disc implant as defined in independent claim 1. Advantageous further embodiments are recited in the dependent claims.

Artificial spinal disc implants are provided.

In one aspect, an artificial spinal disc implant constructed and arranged for implantation between adjacent vertebrae in a living being is provided. The spinal disc implant comprises a body; and a first end plate provided with the body. The first end plate includes a first fixation element and a second fixation element. The first end plate has an anterior side and a posterior side. A total volume of the first fixation element is greater than a total volume of the second fixation element and the first fixation element is closer to the posterior side than the second fixation element.

In one embodiment, an artificial spinal disc implant constructed and arranged for implantation between adjacent vertebrae in a living being is provided. The spinal disc implant comprises a body; and, a first end plate provided with the body. The first end plate including a posterior side, an anterior side and a reference point defined at the posterior side along a mid-line that extends from the posterior side to the anterior side. The first end plate includes a first arrangement of at least one fixation element contacting a first arc having a first radial distance from the reference point and sweeping over a 90 degree angle to the mid-line. The first arrangement having a first total fixation element volume. The first end plate includes a second arrangement of at least one fixation element contacting a second arc having a second radial distance from the reference point and sweeping over a 90 degree angle to the mid-line. The second arrangement having a second total fixation element volume. The first total fixation element volume is greater than the second total fixation element volume and the second radial distance is greater than the first radial distance.

In one embodiment, an artificial spinal disc implant constructed and arranged for implantation between adjacent vertebrae in a living being is provided. The spinal disc implant comprises a first end plate provided with the body. The first end plate includes a reference point defined at a posterior side of the first end plate and a first arrangement of at least one fixation element. The first arrangement has a first total fixation element volume, and each fixation element within the first arrangement is approximately a first minimum distance from the reference point. The first end plate includes a second arrangement of at least one fixation element. The second arrangement has a second total fixation element volume, and each fixation element within the second arrangement is approximately a second minimum distance from the reference point. The first total fixation volume is greater than the second total fixation volume and the first minimum distance is less than the second minimum distance.

For purposes of clarity, not every component is labeled in every figure. Nor is every component of each embodiment of the invention shown where illustration is not necessary to allow those of ordinary skill in the art to understand the invention. All patent applications and patents incorporated herein by reference are incorporated by reference in their entirety. In case of conflict, the present specification, including definitions (if any), will control.

### Brief Description of the Drawings

FIG. 1A shows a spinal disc implant in accordance with embodiments of the invention;
FIG. 1B shows a side view of the spinal disc implant of FIG. 1A;
FIG. 1C is a top view of the spinal disc implant of FIG. 1A;
FIG. 1D is another top view of the spinal disc implant of FIG. 1A;
FIG. 2 is a top view of another spinal disc implant in accordance with embodiments of the invention;
FIG. 3 is a top view of another spinal disc implant in accordance with embodiments of the invention;
FIG. 4A is a cross-sectional schematic of fixation elements of a spinal disc implant and corresponding recesses on a vertebrae in accordance with embodiments of the invention;
FIG. 4B is a cross-sectional schematic of a fixation element of a spinal disc implant in accordance with embodiments of the invention;
FIGS. 5A-5F shows arrangement of fixation elements of the spinal disc implant in accordance with embodiments of the invention

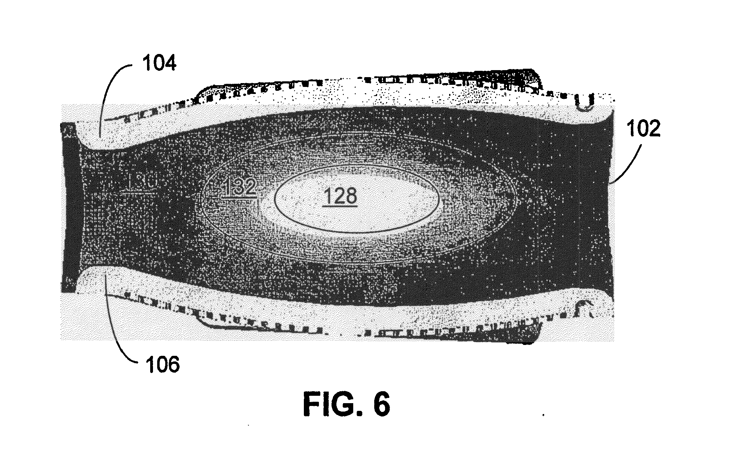

FIG. 6 is a cross-section of a spinal disc implant in accordance with embodiments of the invention;

### Detailed Description

An artificial spinal disc implant is provided that may be implanted between adjacent vertebrae in the spine to replace, repair or augment a natural spinal disc. The spinal disc implant may be characterized by one or more biomechanical properties that approximate those of a natural spinal disc. The implant is also designed to be sufficiently secured to the vertebrae so that it may function in the body for long time periods.

The disc implant may include one or more end plates. The end plates may have an outer surface that includes fixation elements that are designed to fit into corresponding features (e.g., recesses) on the vertebrae to secure the disc between the vertebrae. The fixation elements may be arranged and configured to provide a greater resistance to forces that act to dislodge the disc from its position between the vertebrae. For example, the fixation elements may be designed to have certain preferred dimensions and/or may be positioned in certain preferred arrangements on the end plates, as described further below. The fixation elements may also help to provide the end plates of the disc with a desirable stiffness.

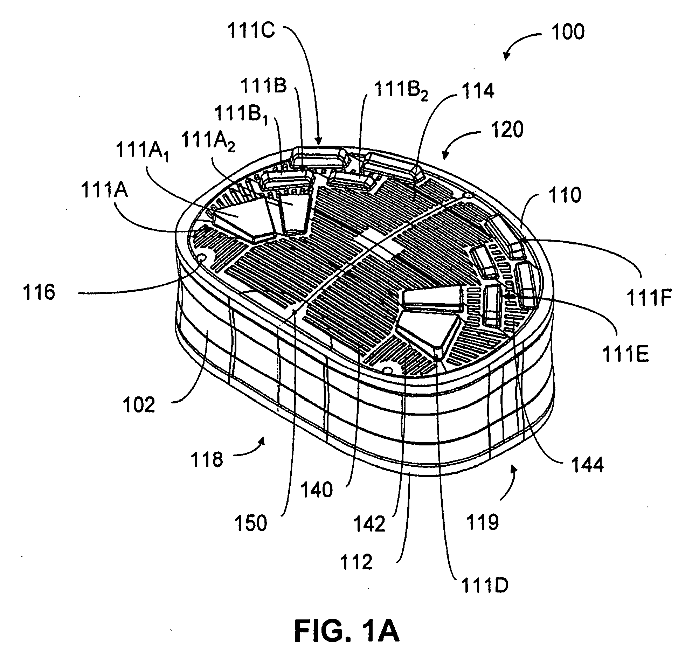

FIGS. 1A-1D show an artificial spinal disc implant 100 in accordance with embodiments of the invention. Disc 100 includes a body 102 having an upper surface 104, a lower surface 106 and a sidewall 108. The disc includes a first end plate 110 and a second end plate 112 which, in these illustrative embodiments, are on upper surface 104 and lower surface 106, respectively. It should be understood, however, that the end plates may be provided on the disc in other manners. As described further below, the illustrated end plates include arrangements of fixation elements 111 which aid in securing the disc to adjacent vertebrae when implanted. The end plates also include a series of surface features 114 designed to enhance bone growth on the end plate and integration of the implanted disc within the body. The disc may also include one or more radiopaque markers 116 which can be used to identify disc position for ensuring proper placement.

It should be understood that the spinal disc shown in FIGS. 1A-1D is an illustrative embodiment of the invention and that the invention is not limited to this or other embodiments shown in the figures and/or described in the Detailed Description.

End plates 110 and 112 are shown as including a series of arrangements of fixation elements 111. The fixation elements can be used to secure the disc in a proper position when implanted. For example, fixation elements may interact with portions of the vertebrae to fixate the disc. In some embodiments, the fixation elements fit into one or more corresponding recesses that may be formed in the vertebrae. When positioned in the recess(es), fixation elements may be constructed and arranged to resist shear and rotation.

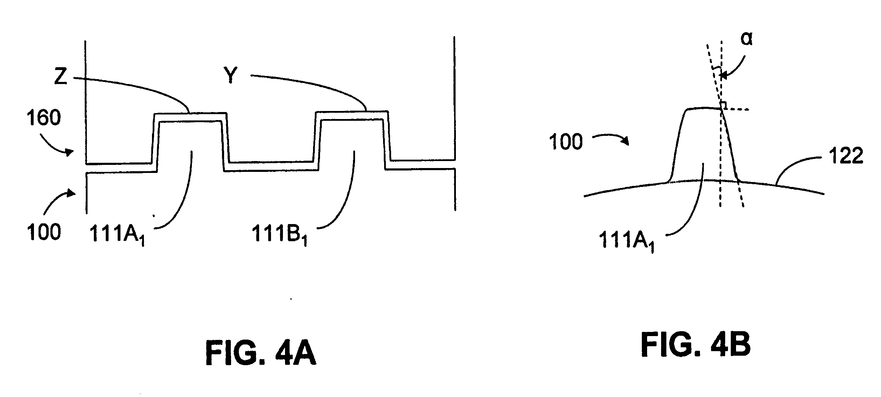

As an illustrative example, FIG. 4A shows a cross-sectional schematic view of a spinal disc implant 100 that is readily secured to a neighboring vertebrae 160. In this example, fixation elements 111A₁ and 111B₁ of disc implant 100 may suitably fit into recesses Z and Y, respectively, that are formed in vertebrae 160. The recesses, for example, may be formed using a suitable instrument. It should be understood that it is not a necessary aspect for recesses to be formed in the vertebrae to secure a disc. Indeed, a disc implant may be suitably secured using a natural contour of the vertebrae.

It can also be appreciated that the surface of end plates 110 or 112 and fixation elements 111 may be shaped in any suitable configuration. In some embodiments, the surface may be substantially straight, as shown in FIG. 4A. In other embodiments, the surface may be curved or textured. As an example, FIG. 4B depicts a cross-sectional schematic view of another embodiment of a fixation element 111A₁ on a spinal disc implant 100. In this example, fixation element 111A₁ is situated on a dome-shaped region 122 of implant 100, given by the curvature of the surface along the base of the fixation element. In addition, transitions from the base upon which fixation element 111A₁ is situated to the element itself may be characterized by any appropriate feature. In FIG. 4B, the base from the dome curves up into fixation element 111A₁, while fixation element 111A₁, itself, exhibits slightly curved features as well. It should be understood that any textured aspect (not shown) of fixation elements or end plate surfaces may be incorporated or any combination of that mentioned previously thereof in addition.

Moreover, fixation elements may be shaped so as to incorporate a draught angle α. In one aspect, a draught angle allows for easy removal of the device from the tool from which it is manufactured along with easy insertion of the device into regions of interest along vertebrae. FIG. 4B shows a draught angle, as described. In various embodiments, draught angle α may fall within a range between 0 and 10 degrees.

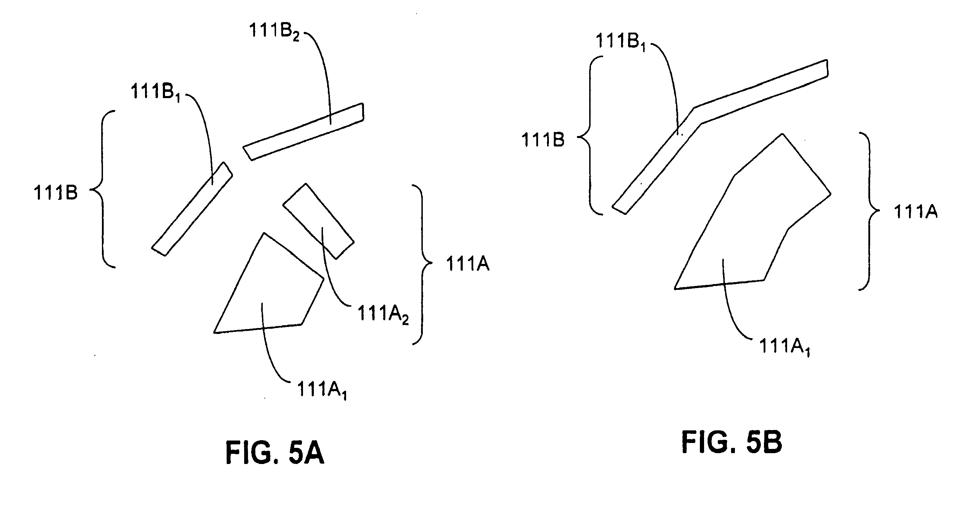

In the embodiment shown in FIG. 1A, the fixation elements are provided in a series of arrangements (or groupings) A, B, C, D, E, F of one or more fixation elements. On the figures, the fixation elements in arrangement A are labeled as 111A₁ and 111A₂; the fixation elements in arrangement B are labeled as 111B₁ and 111B₂; and the like. In the embodiment shown, each arrangement has two fixation elements. However, it should be understood that other arrangements are possible including any suitable number of fixation elements. Additional arrangements are described below and shown in FIGS. 5A-5F.

Though the disc shown in FIG. 1A has six arrangements (A-F) of fixation elements, it should also be understood that the disc may include any suitable number of arrangements. For example, the disc may include one, two, three, four, or more, arrangement(s) of fixation elements.

According to the invention, each fixation element in an arrangement is aligned with one another as shown in FIGS. 1A-1D. The alignment is the result of each fixation element in an arrangement being positioned along (i.e., in contact with) an arc defined from a reference point (e.g., 150) on the end plate surface. An arc, as described herein, refers to a curve that is defined by a reference point (e.g., 150), a radial distance, and a sweep angle. In this respect, the reference point may be defined at any suitable location (e.g., 150), the radial distance may be any appropriate length, and the sweep angle may range anywhere from 0 to 360 degrees. The reference point (e.g., 150) may be, for example, at a posterior side 118 of the disc on a midline that extends from the posterior side to an anterior side 120. The reference point may be a center of rotation for arrangements of fixation elements. In the embodiment shown, reference point 150 acts as a common center of rotation for arrangements of fixation elements 111A, 111B, 111C, 111D, 111E, and 111F. The reference point may be, for example, a dimple, or a cavity. However, it should be understood that the reference point and the arc(s) need not have be a physical structure on the disc. In some cases, the minimum distance between each element in an arrangement and the reference point (e.g., 150) may be approximately the same.

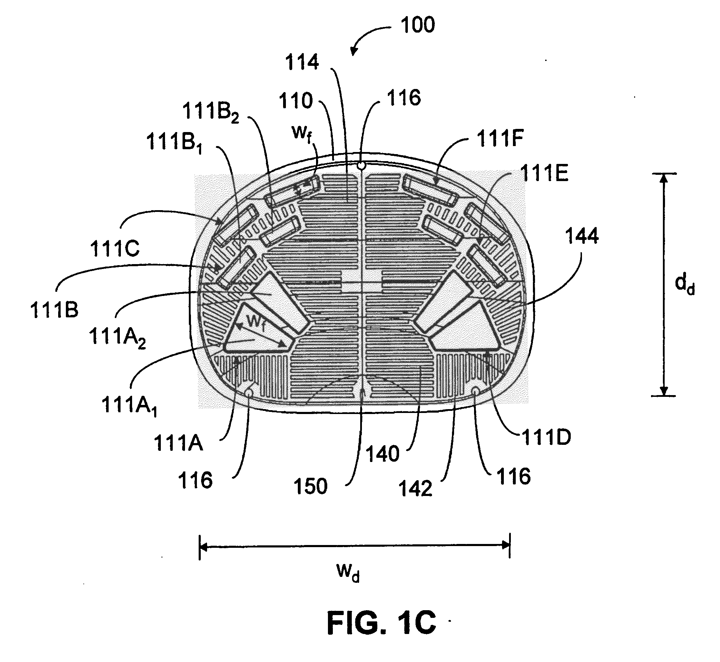

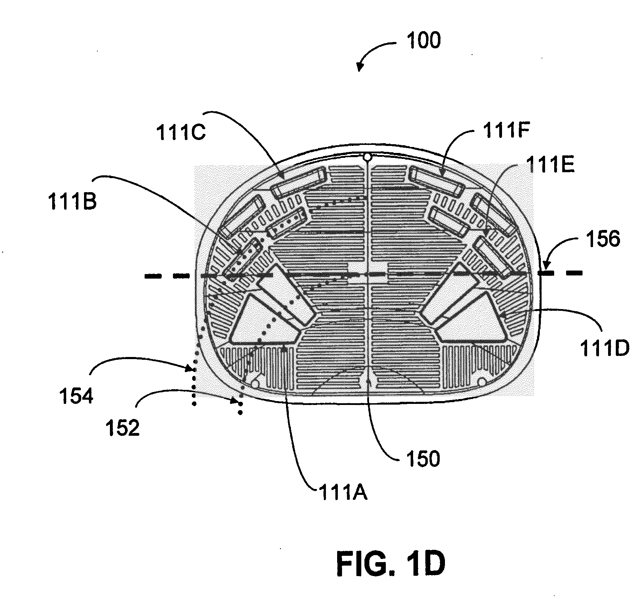

As shown in FIG. 1A, 1C, and 1D the distance between the arc 152 that comes across arrangement A and reference point 150 is less than the distance between the arc 154 that comes across arrangement B and reference point 150. In some embodiments, and as shown in FIG. 1D, more than one arrangement may be on the same arc. For example, arrangements A and D may lie along the same arc (not shown) sweeping over an angle of approximately 180 degrees. In other embodiments, as shown in FIG. 1D, for a given reference point and radial distance, an arc 152 that sweeps over an angle of 90 degrees may include an arrangement 111A. In further embodiments, for a given reference point and radial distance, an arc that sweeps over a smaller angle (not shown), for example, 30 degrees, may also include an arrangement of fixation elements.

Each arrangement has a total fixation element volume which is defined as the sum of the volumes of each fixation element in the arrangement. The volume of a fixation element is the total volume that protrudes from the end plate surface. For example, fixation elements 111A,..., 111F have a volume generally defined, in part, by the top surface of the fixation element and a side surface of the fixation element. Side surfaces may have any appropriate height, contributing to the overall volume of fixation elements. Similarly, top surfaces may exhibit any suitable horizontal surface, contributing to the overall volume of fixation elements as well. It should be understood that when defining a fixation element by an arc, the volume of the fixation element is determined by the volume of the actual fixation element, and not the volume that is swept out by the plane of the arc.

In one aspect, the total fixation element volume is designed to provide desirable disc properties including a greater stiffness and/or greater resistance to forces on the end plates of the disc. In some embodiments, the total fixation element volume of one arrangement closer to posterior side 118 may be greater than the total fixation element volume of one arrangement further from the posterior side. Such a configuration has been shown to be particularly effective in increasing end plate stiffness in certain disc implant designs. For example, the minimum distance between the arrangement having a greater total fixation element volume and the posterior side is less than the minimum distance between the arrangement having a lower total fixation element volume and the posterior side. In this regard, the posterior side 118 can be understood to include the substantially straight edge of the disc along with curved portions that transition to the side edges 119.

In some cases, and as shown, the arrangement(s) (e.g., A and D) having the greatest total fixation element volume are positioned closer to the posterior side than each of the other arrangements. As shown in the embodiment in FIGS. 1A-1D, arrangement A of fixation elements has a greater total fixation element volume than arrangement B and C. Similarly, arrangement D has a greater total fixation element volume than arrangement E and F.

In some embodiments, the total fixation element volume on a posterior half of the disc is greater than the total fixation element volume on an anterior half of the disc. As understood herein, shown in FIG. 1D, coronal line 156 is meant to define the anterior half and the posterior half. In this regard, the anterior half includes the portion of the device that is anterior to coronal line 156 and the posterior half includes the portion of the device that is posterior to coronal line 156.

In some embodiments, an arrangement (e.g., A) positioned on an arc having a shorter radial distance from a reference point (e.g., 50) may have a larger total fixation element volume than an arrangement (e.g., B, C) positioned on an arc having a longer radial distance from that reference point. The arc, for example, may sweep over a 90 degree angle to a mid-line that extends from posterior side to anterior side. Such a configuration has also been shown to be particularly effective in increasing end plate stiffness of certain disc implant designs.

It should be understood that, in some embodiments and as shown, more than one arrangement may have a similar total fixation element volume as one or more other arrangements (e.g., A and D; or B and C and E and F). In some cases, the total fixation element volume of one arrangement (e.g., A) may be greater than the total fixation element volume of another arrangement (e.g., B or C).

The absolute value of the total fixation element volume may depend on the overall size of the disc which can depend on the size of the natural disc being replaced, repaired or augmented. In some embodiments, the arrangement having the larger total fixation element volume (e.g., A) may have a total fixation element volume between approximately 10 mm³ and approximately 100 mm³; in other embodiments, between approximately 15 mm³ and approximately 60 mm³. It can be appreciated that the volume of fixation elements that may be in closer proximity to the posterior edge may be designed to increase as the surface area of the corresponding vertebral footprint increases.

In another aspect, the arrangement having the smaller total fixation element volume (e.g., B and C) for a given disc may have a total fixation element volume between approximately 2 mm³ and approximately 50 mm³; in other embodiments, between approximately 5 mm³ and approximately 30 mm³.

In addition, the total fixation element volume of one arrangement (e.g., A) may be greater than 1.5 times the total fixation element volume of another arrangement (e.g., B, C); in some cases, the total fixation element volume of one arrangement (e.g., A) may be greater than 2 times the total fixation element volume of another arrangement (e.g., B, C); in some cases, the total fixation element volume of one arrangement (e.g., A) may be between 1 and 3 times or, between 1 and 6 times, the total fixation element volume of another arrangement (e.g., B, C).

It should also be understood that, in some aspects, the above-noted comparisons with respect to the total fixation element volumes between arrangements may also translate to similar comparisons with respect to the total fixation element surface area between arrangements, for example, if the thickness and topography of the fixation elements are relatively constant. Thus, the total fixation element surface area of one arrangement (e.g., A) may be greater than 1.5 times the total fixation element surface area of another arrangement (e.g., B, C); in some cases, the total fixation element surface area of one arrangement (e.g., A) may be greater than 2 times the total fixation element surface area of another arrangement (e.g., B, C); in some cases, the total fixation element surface area of one arrangement (e.g., A) may be between 1 and 6 times (or between 1 and 5 times) the total fixation element surface area of another arrangement (e.g., B, C).

The total fixation element surface area may depend on the overall size of the disc which can depend on the size of the natural disc being replaced, repaired or augmented. In this respect, fixation element surface may be related to fixation element volume. As used herein, fixation element surface area does not include texturing effects of the fixation elements. That is, the fixation element surface area relates to a substantially planar surface without a significant degree of texturing which would provide an artificial increase in overall surface area. In some embodiments, the arrangement having the larger total fixation element surface area (e.g., A) may have a total fixation element surface area between approximately 20 mm² and approximately 300 mm²; in other embodiments, between approximately 30 mm² and approximately 200 mm².

For another aspect, the arrangement having the smaller total fixation element surface area (e.g., B and C) for a given disc may have a total fixation element surface area between approximately 10 mm² and approximately 100 mm²; in other embodiments, between approximately 30 mm² and approximately 70 mm².

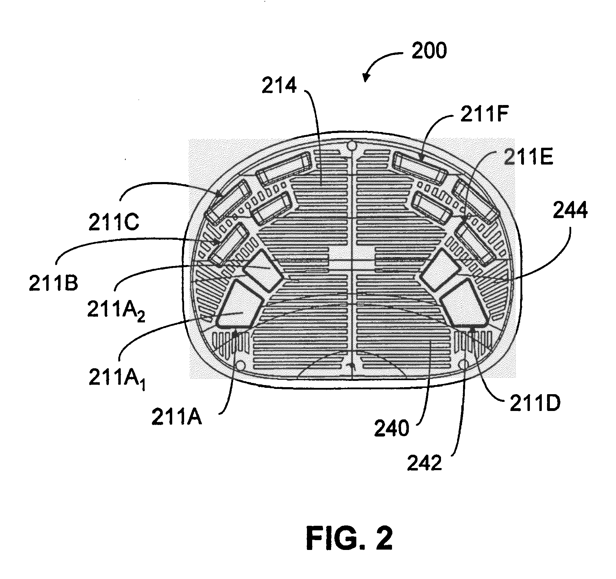

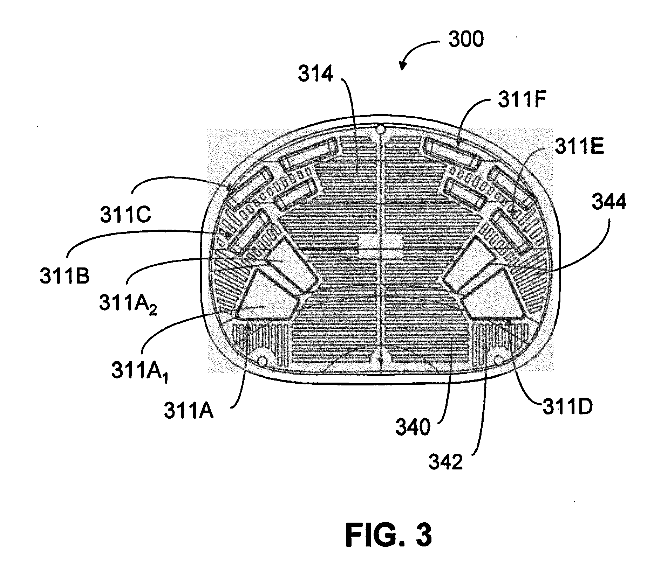

Additional embodiments of discs are shown in FIGS. 2 and 3. In one example shown in FIG. 2, the relative difference between volumes of arrangements of fixation elements 211 A, 211 B, and 211C is different than that of the relative difference between volumes of arrangements of fixation elements 111 A, 111B, and 111C. In this regard, comparing device 100 to device 200 as an example, the ratio between the volume of the first arrangement of fixation elements 111A to the second arrangement of fixation elements 111B is greater than the ratio between the volume of the first arrangement of fixation elements 211A to the second arrangement of fixation elements 211B. In this case, the volumes of second and third arrangements of fixation elements 111B and 111C of device 100 is substantially similar to the volumes of second and third arrangements of fixation elements 211B and 211C of device 200. It should be understood that the volumes of the arrangements of fixation elements for any device may be designed according to conditions appropriate for suitable insertion of the device into the body between proper vertebrae.

FIG. 3 depicts another embodiment of a disc implant device 300 with arrangements of fixation elements 311 A, 311 B, and 311C. In this example, the total volume of the first arrangement of fixation elements 311 A is in between that of the volumes of first and second arrangements of fixation elements 111A and 211A. Similarly to the example shown in FIG. 2, the volumes of second and third arrangements of fixation elements 311 B and 311C of device 300 may be substantially similar to the volumes of second and third arrangements of fixation elements 111B and 111C of device 100. In this regard, depending on the loading conditions that are experienced by the particular implant location, the volume may be designed accordingly.

In some embodiments, each fixation element in an arrangement may fit into a single corresponding recess within the vertebrae. For example, with reference to FIG. 4A, all of the fixation elements in arrangement A may fit into a recess Z in the vertebrae; and, all of the fixation elements in arrangement B may fit into a recess Y in the vertebrae; and, the like. However, it should be understood that not all fixation elements in an arrangement need to fit into the same recess in all embodiments.

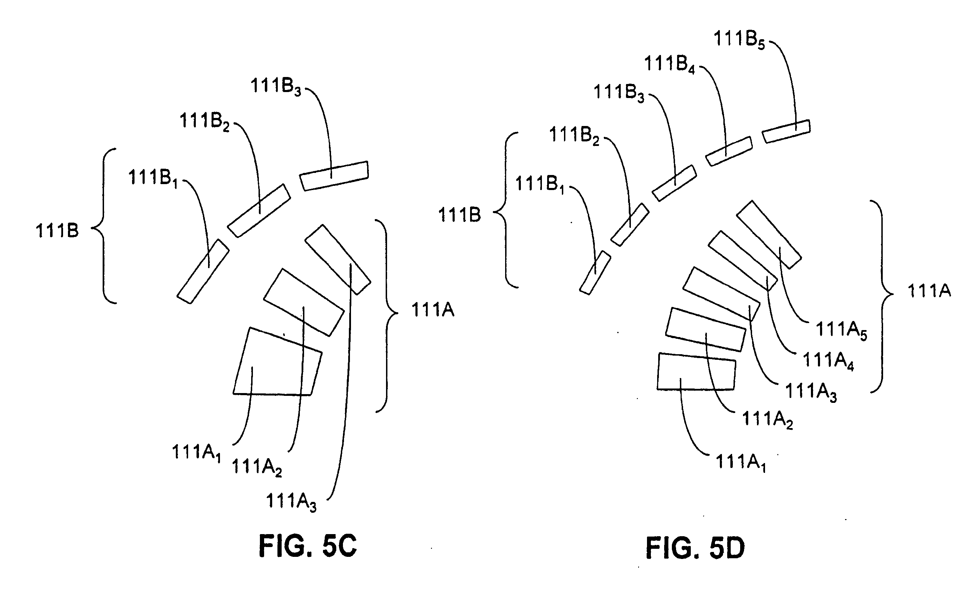

It should be understood that a wide variety of fixation elements are possible. FIGS. 5A-5F show non-limiting examples. As depicted in the figures for various embodiments, two fixation elements exist for each arrangement 111A and 111B, as shown in FIG. 5A. In this embodiment, the first arrangement 111A has two fixation elements 111A₁ and 111A₂. The second arrangement 111B also has two fixation elements 111B₁ and 111B₂. It should be understood that arrangements of fixation elements are not limited to two fixation elements per arrangement. As shown, as illustrative embodiments, in FIGS. 5B-5F any suitable number of fixation elements may be incorporated in each arrangement. In some embodiments, each arrangement 111A and 111B may include a single fixation element 111A₁ and 111B₁, respectively, as depicted in FIG. 5B. In other embodiments shown in FIGS. 5C and 5D, arrangements may include three or more fixation elements. FIG. 5C shows a first arrangement 111A having three fixation elements 111A₁, 111A₂, and 111A₃. Similarly, second arrangement 111B includes three fixation elements 111B₁, 111B₂, and 111B₃. FIG. 5D shows a first arrangement 111 A having five fixation elements 111A₁, 111A₂, 111A₃, 111A₄, and 111A₅. Additionally, second arrangement 111B includes five fixation elements with volumes 111B₁, 111B₂, 111B₃, 111B₄, and 111B₅. In this regard, the number of fixation elements per arrangement is not meant to be limited to that which is described herein.

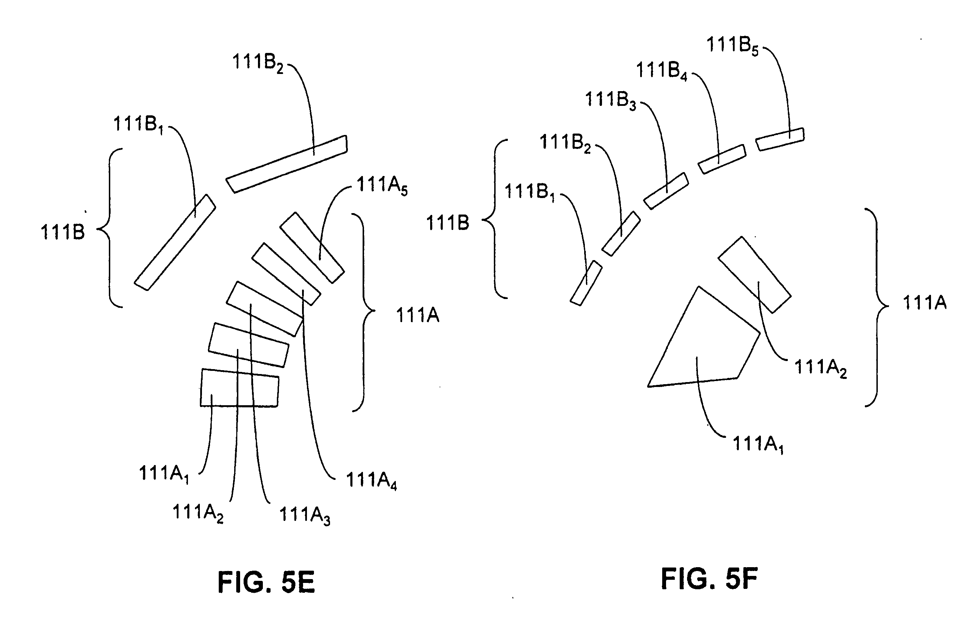

In addition, the number of fixation elements in an arrangement are not limited to the number of fixation elements in an adjacent arrangement. In one embodiment, FIG. 5E depicts an example where the first arrangement 111A includes five fixation elements 111A₁, 111A₂, 111A₃, 111A₄, and 111A₅ and the second arrangement 111B includes two fixation elements 111B₁ and 111B₂. In another embodiment, FIG. 5F shows an example where the first arrangement 111A includes two fixation elements 111A₁ and 111A₂ and the second arrangement 111B includes five fixation elements 111B₁, 111B₂, 111B₃, 111B₄, and 111B₅.

Fixation elements may also be formed of any suitable shape. In FIG. 5A, the first arrangement of fixation elements 111A includes parallelogram and quadrilateral shaped elements. The second arrangement of fixation elements 111B includes quadrilateral elements that are substantially rectangular in shape. It can be appreciated that the fixation elements are not required to be quadrilateral. Indeed, fixation elements may have any suitable number of sides. As a non-limiting example, FIG. 5B shows fixation elements that have more than four sides. In various embodiments, fixation elements may also include curved or rounded edges that may or may not be separated by corners. In addition, fixation elements that cover a large surface area tend to take up large volume spaces as well.

As shown in FIGS. 1A-1D, the width (w_{f}) of the fixation element(s) in the arrangement having the larger total fixation element volume may be greater than the width of the fixation element(s) in the arrangement having the smaller total fixation element volume. For example, the width of the fixation element in the arrangement having the larger total fixation element volume may be at least two times, at least three times, or at least four times the width of the fixation element(s) in the arrangement having the smaller total fixation element volume.

The fixation elements may be formed of the same material as other portions of the end plate. As described further below, suitable materials include polymeric materials such as polyurethane materials. In some embodiments, the fixation elements are formed integrally (e.g., in the same process) with other portions of the end plate. However, it is possible for the fixation elements to be formed separately and/or from a different material than other portions of the end plate.

It should be understood that other suitable types of fixation elements and/or fixation element arrangements are also within the scope of that described herein.

End plates 110 and 112 also can include surface features 114 which are designed to enhance bone growth on the end plates and integration of the implant disc within the human body. In the illustrative embodiment, surface features 114 include macro-texture features (e.g., protrusions that may be smaller than previously described fixation elements) that form a series of inter-connected channels defined in the outer surface of the end plates. The channels, for example, may have a width of between 100 microns and 750 microns (e.g., 400 microns). The macro-texture features may also be protrusions having a width of between 200 microns and 400 microns (e.g., 300 microns) and a height of between 100 microns and 300 microns (e.g., 200 microns).

Surface features 114 may include macro-texture features 140, 142, and 144 that may be arranged in any suitable orientation. In the embodiments depicted, such as in FIG. 1C, macro-texture features 140 are oriented in a substantially horizontal direction. As shown in FIG. 1C, macro-texture features 142 are oriented in a substantially vertical direction. Macro-texture features 144 are oriented in an angled direction.

It should be understood that macro-texture features are not required to conform rigidly to a particularly orientation or direction, but may be designed in any way. In this respect, macro-texture features do not have to be oriented in a substantially vertical or horizontal direction. Indeed, all macro-texture features could be angled or arranged into shapes (similar to fixation elements).

In various aspects, macro-texture features 140, 142, and 144 may be arranged to aid in increasing bending stiffness of the disc. In some embodiments, macro-texture features 140 and 142 may be substantially perpendicular to one another. In other embodiments, macro-texture features 140 may extend from a midline that runs from the posterior of the disc 118 to the anterior of the disc 120. In this regard, macro-texture features 140 may extend to fixation element arrangements on the end plate. In further embodiments, macro-texture features 142 may be located predominantly around radiopaque markers 116 in close proximity to a first arrangement of fixation elements 111 A. In more embodiments, macro-texture features 144 may be located between the first arrangement of fixation elements 1111A and the second arrangement of fixation elements 111B. Macro-texture features 144 may also be located between the second arrangement of fixation elements 111 B and the third arrangement of fixation elements 111C.

For embodiments shown in FIGS. 2 and FIG. 3, macro-texture features 214 and 314 may also be provided. In this regard, macro-texture features of varying orientations, similar to that described above, may also be provided. Such orientations include substantially horizontal macro-texture features 240 and 340, substantially vertical macro-texture features 242 and 342, and angled macro-texture features 244 and 344.

Regarding the embodiments depicted in FIGS. 1C and 2, vertically oriented macro-texture features 142 take up a larger percentage of the surface area on the respective endplate as compared to vertically oriented macro-texture features 242. As a result, horizontally oriented macro-texture features 140 take up a smaller percentage of the surface area on the respective endplate as compared to horizontally oriented macro-texture features 240. In these cases, the percentage of surface area covered by macro-texture features 144 and 244 are approximately equal.

With respect to the embodiment shown in FIG. 3, percentage of surface area on the respective endplate that is taken up by the vertically oriented macro-texture features 342 is in between that of the percentage of surface area on the respective endplates taken up by vertically oriented macro-texture features 142 and 242 of devices 100 and 200, respectively. In this regard, depending on the loading condition that is experienced by the particular implant location, the relative amount of surface area taken up by a particular orientation of macro-texture features may be modified as appropriately desired.

In general, the end plates may be formed of any suitable material including rigid polymeric materials such as certain polyurethane materials (e.g., polyurethane polycarbonate materials). As noted above, in some embodiments, outer surfaces of the end plates are formed of a material having properties selected to complement or match that of the cancellous bone adjacent end plate surfaces when the disc is implanted. The end plate material (e.g., polyurethane material) may be appropriately formulated to provide such desirable properties. For example, the end plate material may have a hardness or compressive modulus similar to that of cancellous bone and less than those of certain conventional metal end plate materials (e.g., titanium, cobalt-chrome alloys). For example, the material may have a hardness of between 50 Shore D and 100 Shore D, or between 70 Shore D and 90 Shore D. Shore hardness may be measured using procedures and instruments known to those of ordinary skill in the art. For example, suitable techniques for measuring Shore hardness for polymeric material are described in ASTM D2240. End plates having outer surfaces formed of materials having such hardness values can lead to minimal stress shielding and does not enhance degenerative conditions in regions of the spine around the implant.

In some embodiments, the end plates are formed entirely from a material having the above-noted hardness values. In these embodiments, the end plates may have a unitary construction. The end plates may also be formed of more than one material with the outer surface of the end plates being formed of a material having the above-noted hardness values and other portions of the end plates being formed of one or more other materials (including materials which may not have the above-noted hardness values).

It should be understood that not all embodiments include end plates having outer surfaces formed of materials having the above-noted hardness ranges. Also, in other embodiments, one of the end plates may have an outer surface formed of a material having the above-noted hardness values while the other end plate may not.

End plates 110 and 112 may have a dome-shape outer surface. As shown, end plates 110 and 112 have a dome-shaped region 122 that extends vertically from a flat portion 124. Though, it should be understood, that in other embodiments the entire outer surface of the end plate may be dome-shaped. The dome-shaped region may have dimensions selected to be compatible with the morphology of vertebral bodies. The domed-shape region may also facilitate the implant procedure. For example, the dome-shaped region may have a maximum dome height of between 0.75 mm and 3.0 mm, or between 0.75 mm and 1.5 mm. The dome-shaped region may also be characterized by having a width (w_{d}) and a depth (d_{d}). The width-to-depth ratio, for example, may be between 1.1 and 1.8 (e.g., 1.4). In some embodiments, it may be preferable that the fixation elements have a height that does not exceed the height of a dome-shaped region 122. Such a construction can facilitate proper placement of the disc within the vertebrae. For example, the fixation elements may have a height of less than 1.5 mm.

End plates 110 and 112 may also have micro-texture features (not shown). For example, the micro-texture features may have an average surface roughness (Rₐ) of between 0.1 micron and 10 micron. Average surface roughness (Rₐ) may be measured using procedures and instruments known to those of ordinary skill in the art including surface profilometers. The micro-texture features may also enhance bone growth on the end plates. The features may be configured to encourage/facilitate osteointegration which may make the end plate surface osteoconductive even if the end plate material may not be known as an osteoconductive material.

Outer surfaces of end plates 110 and 112 may also be coated with a suitable material to enhance bone growth. For example, suitable coating materials include osteoconductive materials (e.g., osteoconductive ceramics or osteoconductive polymers), osteophylic materials and bioactive coatings (e.g., bone morphogenic proteins, BMP).

End plates 110 and 112 may be provided with the body in any suitable manner and using any suitable technique. For example, end plates 110 and 112 may be attached to a portion of the body. In some embodiments, the end plates are attached to the body during the manufacturing process as described further below. In these embodiments, the end plates and the body may form an integral (i.e., unitary) piece. That is, there are no distinct interfaces between respective end plates and the body. In these embodiments, the end plates may be attached without the use of a separate adhesive or glue; rather, the end plate material may be chemically bonded directly to the material of the body. For example, when the end plates are formed of polymeric material and the body is formed of polymeric material, chemical (e.g., covalent) bonds may be formed between the polymeric material of the end plate and polymeric material of the body. In some cases, polymeric chains may extend between the polymeric material of the end plate and polymeric material of the body. Eliminating the presence of distinct interfaces between the end plates and the body can enhance durability of the disc since such interfaces can be sites of de-lamination which can lead to failure of the disc.

It should also be understood that some embodiments may involve attaching the end plates to the body using a separate adhesive or glue. In such embodiments, a layer of adhesive or glue, thus, may be formed at the interface between the end plate and the body.

Body 102 of the disc may be formed of any suitable material including biocompatible polymeric materials such as polyurethane materials. It should be understood that polyurethane materials include any polymeric material having a polyurethane component. Such materials may also include other polymeric components such as polycarbonate (e.g., polyurethane polycarbonate materials). Linear and crosslinked polyurethane materials may be suitable. In some embodiments, body 102 may be formed of a single type of polymeric material (e.g., a polyurethane material), as described further below. In embodiments in which the body is formed of only polyurethane material, different portions of the body may comprise polyurethane material having different stoichiometries and/or molecular weights.

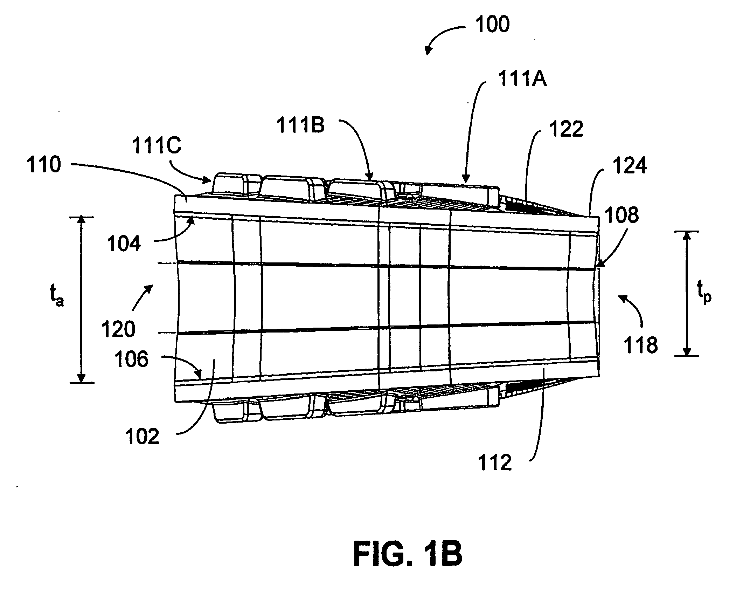

In general, the dimensions of body 102 are selected to be suitable for implantation to replace, repair and/or augment a natural spinal disc. For example, the body may have a width of between about 37 to 47 (e.g., 42 mm); a depth between the posterior side 118 and the anterior side 120 of between about 27 to 37 mm (e.g., 32 mm); and, a thickness of between about 7 mm and 15 mm. In the illustrated embodiments, upper surface 104 and lower surface 106 may be angled so that posterior side 118 has a thickness tₚ that is less than a thickness tₐ of anterior side 120. For example, the angle may be between 6° and 12°. Such a construction may be advantageous for positioning the implant in the spine and/or for biomechanical performance once implanted. As shown in FIG. 1B, sidewall 108 may be tapered toward the center of the body defining a concave surface that forms a curved "waist". The "waist" construction may enhance flexibility of the disc and/or may reduce internal stresses, particularly in response to bending forces. In some cases, stress reduction may be enhanced when the concave portion extends inward a maximum distance (d) (e.g., perpendicular distance from the perpendicular line connecting the outermost edge of the upper and lower end plates) of between 0.5 mm and 5 mm; or, in some embodiments between 0.5 mm and 3 mm. It should be understood that the body may also include straight sidewalls and, thus, does not include a "waist" in all embodiments. Although the curved "waist" illustrated in the figures extends completely around the body, it is also contemplated that a curved waist may be provided at one or more regions around the body.

As noted above, the disc may include one or more radiopaque markers 116 which can be used to identify disc position and to ensure proper placement. In general, radiopaque markers 116 may be formed of any suitable material including those that are visible with x-rays systems and are compatible with the body. Suitable materials for the markers include certain metals (such as titanium, tantalum, gold, tungsten, platinum and mixtures thereof) and polymeric materials loaded with a radio-opacifier (e.g., barium compounds including barium sulfate). The radiopaque markers, for example, may be circular regions. The circular regions may have a diameter of less than 2 mm.

In the illustrative embodiments, three radiopaque markers 116 are arranged to define corners of a triangular shape. As shown, two of the markers are positioned on posterior side 118 of the disc and one on anterior side 120. Such an arrangement enables precise location and orientation of the disc which can ensure proper placement of the implant. Other types and arrangements of radiopaque markers are also possible.

FIG. 5 illustrates an embodiment in which body 102 includes a nucleus region 128 completely surrounded, so as to be encapsulated, by an annulus region 130. Such a body construction has been described in U.S. Patent Application Publication No. 2007/0276492 which is incorporated herein by reference in its entirety and is based on commonly-owned U.S. Patent Application Serial No. 11/431,121, filed on May 9, 2006. Thus, the nucleus region is separated from upper surface 104, lower surface 106 and sidewall 108 of the spinal disc and does not contact the end plates. In certain embodiments, the annulus region may surround the nucleus region to a lesser extent. For example, the annulus region may surround the majority of, but not the entire, surface area of the nucleus region (e.g., between 50% and 90% of the surface area of the nucleus region). For example, part of the top and/or bottom of the nucleus may be covered by the annulus, while certain region or regions of the top and/or bottom of the nucleus are not surrounded by the annulus. Similarly, some, but not, all of the side of the nucleus may be covered by the annulus, while certain region or regions of the side of the nucleus are not surrounded by the annulus.

Nucleus region 128 may have different properties than annulus region 130. The nucleus region may have properties selected to mimic the function of the nucleus pulposus in a natural spinal disc; while, the annulus region may have properties selected to mimic the function of the annulus fibrosis in a natural spinal disc. For example, the nucleus region may be relatively soft and compliant; while, the annulus region may be stiffer and stronger. In particular, the nucleus region may have a Young's modulus that is lower than a Young's modulus within the annulus region.

As noted above, in some embodiments, the annulus region may include a graded portion 132 across which a property, such as Young's modulus, is varied. That is, the Young's modulus changes with distance in a direction across the portion. In some cases, it is preferred that the Young's modulus increase with distance away from the nucleus region. The graded portion 132 may enhance the ability of the disc to absorb and effectively distribute biomechanical loads and stresses. The graded portion can also enable elimination of a distinct interface between the nucleus region and the annulus region, as described further below.

It should be understood that the spinal discs of the invention are not limited to having a body with a nucleus and annulus and/or a graded portion. In general, the discs may have any suitable body including a body having a constant composition.

As noted above, body 102 may be formed of a single type of polymeric material (e.g., a polyurethane material). In embodiments that include a nucleus region and an annulus region, both the nucleus region and the annulus region (including any graded portion that may be present) may be formed of the same type of polymeric material. For example, the nucleus region may be formed of a polyurethane material having a first stoichiometry, while the annulus region may be formed of the same polymeric material composition having a second stoichiometry different than the first stoichiometry. The material in the nucleus region may have a lower molecular weight than the material in the annulus region which results in the difference in stoichiometry. The difference in stoichiometry can also lead to the difference in properties (e.g., Young's modulus) between the nucleus and the annulus region. In embodiments including a portion having graded properties, the stoichiometry of the polymeric material may be similarly graded and can lead to the grade in properties.

In embodiments in which the nucleus region and the annulus region (including graded portion) are formed of the same material, no distinct interfaces are formed between the two regions. As noted above, the absence of distinct interfaces can enhance durability of the disc since such interfaces can be sites of de-lamination during use which can lead to failure of the implant. When the nucleus region and the annulus region are formed of the same material, chemical (e.g., covalent) bonds may be formed between the polymeric material of the nucleus region and the polymeric material of the annulus region. In some cases, polymeric chains may extend between the polymeric material of the nucleus region and the polymeric material of the annulus region.

In one preferred embodiment, spinal disc 100 has a width of between about 37 to 47 (e.g., 42 mm); a depth between posterior side 118 and anterior side 120 of between about 27 to 37 mm (e.g., 32 mm); and, a thickness at posterior side 118 of between about 9 mm and 12 mm. In this embodiment, the thickness at the anterior side is greater than the thickness at the posterior side. For example, the angle defined by the surface extending from the posterior side to the anterior side is between 6° and 12° (e.g., 6°, 9°, 12°). In this embodiment, the body includes a waist defined by a concave portion of the sidewall of the body. The concave portion, for example, extends inward a maximum distance (d) of between 0.5 mm and 5 mm, or 0.5 mm and 3 mm.

In this embodiment, the entire spinal disc 100 (including body and end plates) may be formed of a polyurethane material (e.g., polyurethane polycarbonate materials). Chemical (e.g., covalent) bonds may be formed between polyurethane material of the nucleus region and polyurethane material of the annulus region and polyurethane material of the end plates. In some cases, polymeric chains may extend between polyurethane material in the nucleus region and polyurethane material in the annulus region and polyurethane material in the end plates.

When the entire disc is formed of a single material (e.g., a polyurethane material), there may be no distinct interfaces (e.g., interfaces formed between two separate materials) formed within the entire disc. As noted above, the absence of distinct interfaces can enhance durability of the disc since such interfaces can be sites of de-lamination during use which can lead to failure of the implant. Also, when formed entirely of polymeric materials (e.g., polyurethane materials), the disc may be MRI compatible which is advantageous once implanted.

Spinal disc implants can be designed, as described above, to have properties that are similar to that of a natural spinal disc.

The disc may have an axial stiffness in the range between 1000 N/mm and 3500 N/mm. Axial stiffness is expressed as a force per unit displacement for an applied compressive force which acts perpendicular to the disc mid-plane.

The disc may have a torsional stiffness of between 0.5 Nm/degree and 10 Nm/degree. The torsional stiffness of the disc is described as the force per unit displacement and refers to an isolated implant. Torsional stiffness is calculated by applying a torque using appropriate testing apparatus, through the central loading axis of the implant and recording the angular displacement. The stiffness is subsequently calculated by dividing the applied torque by the angular displacement.

The disc may have a flexural (i.e., bending) stiffness of between 0.5 Nm/degree and 5.0 Nm/degree, between 1.0 Nm/degree and 4.0 Nm/degree, or between 1.0 Nm/degree and 3.0 Nm/degree, for flexion, extension and lateral bending motions (also any motions in between these). The flexural stiffness of the disc is described as the force per unit displacement and, in this case, refers to an isolated implant. One suitable method for measuring flexural stiffness involves the application of a load which is displaced with respect to the central loading axis (i.e., the position at which a point load results in compression only and does not induce any change in angular displacement) of the implant to induce an angular deflection. The moment arm is given by the distance between the central loading axis and loading application point. A goniometer or suitable image capture system may be used to provide real time display of the change in angular displacement with applied load. The bending moment is calculated using simple trigonometry applied to the moment arm and angular displacement. The bending stiffness is subsequently calculated by dividing the applied torque by the angular displacement. It should be noted that the method of calculating the bending stiffness does not take into account the precise location of the center of rotation of the device and also assumes that the center of rotation does not change throughout the load application cycle.

In general, any suitable process may be used to manufacture spinal disc implants of the invention. Suitable processes have been described generally in U.S. Patent Application Publication No. 2007/0276492 and U.S. Patent Application Publication No. US-2006-0167550 based on commonly-owned U.S. Patent Application Serial No. 10/530,919, filed April 8, 2005

Having thus described several aspects of at least one embodiment of this invention, it is to be appreciated various alterations, modifications, and improvements will readily occur to those skilled in the art. Such alterations, modifications, and improvements are intended to be part of this disclosure, and are intended to be within the scope of the invention. Accordingly, the foregoing description and drawings are by way of example only.

## Claims

1. An artificial spinal disc implant constructed and arranged for implantation between adjacent vertebrae in a living being, the spinal disc implant comprising:
a body (102); and
a first end plate (110) provided with the body (102), the first end plate (110) including a first fixation element (111A; 211A; 311 A) and a second fixation element (111B; 211B; 311 B), the first end plate (110) having an anterior side (120) and a posterior side (118), wherein
a total volume of the first fixation element (111A; 211 A; 311 A) is greater than a total volume of the second fixation element (111B; 211B; 311B) and the first fixation element (111A; 211 A; 311 A) is closer to the posterior side (118) than the second fixation element (111B; 211 B; 311B),
the first fixation element (111A; 211 A; 311 A) is in a first arrangement of at least one fixation element, and the second fixation element (111B; 211 B; 311B) is in a second arrangement of at least one fixation element, **characterised in that**
the artificial spinal disc implant further comprises a reference point (150) defined at the posterior side along a mid-line that extends from the posterior side (118) to the anterior side (120),
wherein the first arrangement contacts a first arc (152) having a first radial distance from the reference point (150) and the second arrangement contacting a second arc (154) having a second radial distance from the reference point (150).

2. The implant of claim 1, further comprising a second end plate provided with the body, wherein the second end plate (112) includes a third fixation element and a fourth fixation element, the second end plate having an anterior side and a posterior side,
wherein a total volume of the third fixation element is greater than a total volume of the fourth fixation element and the third fixation element is closer to the posterior side than the fourth fixation element.

3. The implant of claim 1, wherein the first end plate has a dome-shaped region (122).

4. The implant of claim 1, wherein the first fixation element (111A; 211 A; 311A) has a first width and the second fixation element (111B; 211B; 311B) has a second width, and the first width is at least two times the second width.

5. The implant of claim 1, wherein the first end plate (110) further includes:
the reference point (150) defined at the posterior side (118) along the mid-line that extends from the posterior side (118) to the anterior side (120);
the first arrangement of the at least one fixation element (111A; 211 A; 311A) contacting the first arc (152) having the first radial distance from the reference point (150) and sweeping over a 90 degree angle to the mid-line, the first arrangement having a first total fixation element volume; and
the second arrangement of the at least one fixation element (111B; 211 B; 311B) contacting the second arc (154) having the second radial distance from the reference point (150) and sweeping over a 90 degree angle to the mid-line, the second arrangement having a second total fixation element volume,
wherein the second radial distance is greater than the first radial distance.

6. The implant of any one of claims 2 or 5, wherein the first arrangement includes more than one fixation element and the second arrangement includes more than one fixation element.

7. The implant of claim 5, further comprising a second end plate (112) provided with the body (102).

8. The implant of any one of claims 2 or 7, wherein the first end plate (110) and the second end plate (112) are formed integral with the body (102).

9. The implant of any one of claims 2 or 7, wherein the body (102) and the first end plate (110) comprise polymeric material.

10. The implant of any one of claims 1 or 5, wherein the first total fixation element volume is at least two times the second total fixation element volume.

11. The implant of any one of claims 1 or 5, wherein the first total fixation element volume is between 1 and 6 times the second total fixation element volume.

12. The implant of claim 5, wherein the at least one fixation element (111A; 211 A; 311 A) in the first arrangement has a first width and the at least one fixation element (111B; 211 B; 311B) in the second arrangement has a second width, and the first width is at least two times the second width.

13. The implant of claim 1, wherein the first end plate (110) further includes:
the reference point (150) defined at the posterior side (118) of the first end plate (110);
the first arrangement of the at least one fixation element (111A; 211 A; 311 A), the first arrangement having a first total fixation element volume, and each fixation element within the first arrangement being approximately a first minimum distance from the reference point (150); and
the second arrangement of the at least one fixation element (111B; 211B; 311B), the second arrangement having a second total fixation element volume, and each fixation element within the second arrangement being approximately a second minimum distance from the reference point (150),
wherein the first minimum distance is less than the second minimum distance.

## Patentansprüche

1. Künstliches Bandscheibenimplantat, das für eine Implantation zwischen benachbarten Wirbelkörpern in einem Lebewesen ausgeführt und eingerichtet ist, wobei das Bandscheibenimplantat aufweist:
einen Körper (102); und
eine erste Endplatte (110), die mit dem Körper (102) bereitgestellt ist, wobei die erste Endplatte (110) ein erstes Fixationselement (111A; 211A; 311A) und ein zweites Fixationselement (111B; 211B; 311B) aufweist, und die erste Endplatte (110) eine anteriore Seite (120) und eine posteriore Seite (118) aufweist, wobei
ein Gesamtvolumen des ersten Fixationselements (111A; 211A; 311A) größer ist als ein Gesamtvolumen des zweiten Fixationselements (111B; 211B; 311B) und das erste Fixationselement (111A; 211A; 311A) näher an der posterioren Seite (118) ist als das zweite Fixationselement (111B; 211B; 311B), und
das erste Fixationselement (111A; 211A; 311A) in einer ersten Anordnung mit mindestens einem Fixationselement ist und das zweite Fixationselement (111B; 211B; 311B) in einer zweiten Anordnung mit mindestens einem Fixationselement ist, **dadurch gekennzeichnet, dass**
das künstliche Bandscheibenimplantat ferner einen Referenzpunkt (150) aufweist, der bei der posterioren Seite entlang einer Mittellinie definiert ist, die sich von der posterioren Seite (118) zu der anterioren Seite (120) erstreckt,
wobei die erste Anordnung einen ersten Bogen (152) mit einem ersten radialen Abstand von dem Referenzpunkt (150) berührt und die zweite Anordnung einen zweiten Bogen (154) mit einem zweiten radialen Abstand von dem Referenzpunkt (150) berührt.

2. Implantat nach Anspruch 1, ferner mit einer zweiten Endplatte, die mit dem Körper bereitgestellt ist, wobei die zweite Endplatte (112) ein drittes Fixationselement und ein viertes Fixationselement aufweist, und die zweite Endplatte eine anteriore Seite und eine posteriore Seite aufweist,
wobei ein Gesamtvolumen des dritten Fixationselements größer ist als ein Gesamtvolumen des vierten Fixationselements und das dritte Fixationselement näher an der posterioren Seite ist als das vierte Fixationselement.

3. Implantat nach Anspruch 1, bei dem die erste Endplatte einen kuppelförmigen Bereich (122) aufweist.

4. Implantat nach Anspruch 1, bei dem das erste Fixationselement (111A; 211A; 311A) eine erste Breite und das zweite Fixationselement (111B; 211B; 311B) eine zweite Breite aufweist und die erste Breite mindestens zweimal so groß wie die zweite Breite ist.

5. Implantat nach Anspruch 1, bei dem die erste Platte (110) ferner aufweist:
den Referenzpunkt (150), der bei der posterioren Seite (118) entlang der Mittellinie definiert ist, die sich von der posterioren Seite (118) zu der anterioren Seite (120) erstreckt;
wobei die erste Anordnung des mindestens einen Fixationselements (111 A; 211A; 311A) den ersten Bogen (152) mit dem ersten radialen Abstand von dem Referenzpunkt (150) berührt und über einen Winkel von 90° zu der Mittellinie verläuft, und die erste Anordnung ein erstes Gesamtfixationselementvolumen aufweist; und
wobei die zweite Anordnung des mindestens einen Fixationselements (111B; 211B; 311B) den zweiten Bogen (154) mit dem zweiten radialen Abstand von dem Referenzpunkt (150) berührt und über einen Winkel von 90° Winkel zu der Mittellinie verläuft, und die zweite Anordnung ein zweites Gesamtfixationselementvolumen aufweist,
wobei der zweite radiale Abstand größer ist als der erste radiale Abstand.

6. Implantat nach einem der Ansprüche 2 oder 5, bei dem die erste Anordnung mehr als ein Fixationselement aufweist und die zweite Anordnung mehr als ein Fixationselement aufweist.

7. Implantat nach Anspruch 5, ferner mit einer zweiten Endplatte (112), die mit dem Körper (102) bereitgestellt ist.

8. Implantat nach einem der Ansprüche 2 oder 7, bei dem die erste Endplatte (110) und die zweite Endplatte (112) integral mit dem Körper (102) ausgebildet sind.

9. Implantat nach einem der Ansprüche 2 oder 7, bei dem der Körper (102) und die erste Endplatte (110) ein Polymermaterial aufweisen.

10. Implantat nach einem der Ansprüche 1 oder 5, bei dem das erste Gesamtfixationselementvolumen mindestens zweimal so groß ist wie das zweite Gesamtfixationselementvolumen.

11. Implantat nach einem der Ansprüche 1 oder 5, bei dem das erste Gesamtfixationselementvolumen zwischen ein und sechs mal größer als das zweite Gesamtfixationselementvolumen ist.

12. Implantat nach Anspruch 5, bei dem das mindestens eine Fixationselement (111A; 211A; 311A) bei der ersten Anordnung eine erste Breite aufweist und das mindestens eine Fixationselement (111B; 211B; 311B) bei der zweiten Anordnung eine zweite Breite aufweist, und die erste Breite mindestens zweimal so groß ist wie die zweite Breite.

13. Implantat nach Anspruch 1, bei dem die erste Endplatte (110) ferner aufweist:
den Referenzpunkt (150), der bei der posterioren Seite (118) der ersten Endplatte (110) definiert ist;
die erste Anordnung des mindestens einen Fixationselements (111A; 211A; 311A), wobei die erste Anordnung ein erstes Gesamtfixationselementvolumen aufweist und jedes Fixationselement in der ersten Anordnung näherungsweise mit einem ersten minimalen Abstand von dem Referenzpunkt (150) entfernt ist; und
die zweite Anordnung des mindestens einen Fixationselements (111B; 211B; 311B), wobei die zweite Anordnung ein zweites Gesamtfixationselementvolumen aufweist und jedes Fixationselement in der zweiten Anordnung näherungsweise mit einem zweiten minimalen Abstand von dem Referenzpunkt (150) entfernt ist,
wobei der erste minimale Abstand geringer ist als der zweite minimale Abstand.

## Revendications

1. Implant de disque intervertébral artificiel construit et adapté pour une implantation entre des vertèbres adjacentes d'un être vivant, l'implant de disque intervertébral comprenant :
un corps (102) ; et
une première plaque d'extrémité (110) prévue avec le corps (102), la première plaque d'extrémité (110) comprenant un premier élément de fixation (111A ; 211A ; 311 A) et un deuxième élément de fixation (111B ; 211 B ; 311 B), la première plaque d'extrémité (110) ayant un bord antérieur (120) et un bord postérieur (118), dans lequel
un volume total du premier élément de fixation (111A ; 211A ; 311A) est plus grand qu'un volume total du deuxième élément de fixation (111B ; 211B ; 311B) et le premier élément de fixation (111A ; 211A ; 311a) est plus proche du bord postérieur (118) que le deuxième élément de fixation (111B ; 211 B ; 311 B),
le premier élément de fixation (111A ; 211A ; 311A) est dans un premier agencement d'au moins un élément de fixation, et le deuxième élément de fixation (111B ; 211 B ; 311B) est dans un deuxième agencement d'au moins un élément de fixation,
**caractérisé en ce que** l'implant de disque intervertébral comprend en outre un point de référence (150) défini au niveau du bord postérieur (118) sur une ligne médiane qui s'étend depuis le bord postérieur (118) jusqu'au bord antérieur (120),
dans lequel le premier agencement intersecte un premier arc (152) ayant une première distance radiale par rapport au point de référence (150) et le deuxième agencement intersecte un deuxième arc (154) ayant une deuxième distance radiale par rapport au point de référence (150).

2. Implant selon la revendication 1, comprenant en outre une deuxième plaque d'extrémité prévue avec le corps, dans lequel la deuxième plaque d'extrémité (112) comprend un troisième élément de fixation et un quatrième élément de fixation, la deuxième plaque d'extrémité ayant un bord antérieur et un bord postérieur,
dans lequel un volume total du troisième élément de fixation est plus grand qu'un volume total du quatrième élément de fixation et le troisième élément de fixation est plus proche du bord postérieur que le quatrième élément de fixation.

3. Implant selon la revendication 1, dans lequel la première plaque d'extrémité a une région en forme de dôme (122).

4. Implant selon la revendication 1, dans lequel le premier élément de fixation (111A ; 211 A ; 311 A) a une première largeur et le deuxième élément de fixation (111B ; 211B ; 311B) a une deuxième largeur, et la première largeur est au moins deux fois plus grande que la deuxième largeur.

5. Implant selon la revendication 1, dans lequel la première plaque d'extrémité (110) comprend en outre :
le point de référence (150) défini au niveau du bord postérieur (118) sur la ligne médiane qui s'étend depuis le bord postérieur (118) jusqu'au bord antérieur (120),
le premier agencement dudit au moins un élément de fixation (111A ; 211A ; 311A) intersectant le premier arc (152) ayant la première distance radiale par rapport au point de référence (150) et s'étendant sur une plage angulaire de 90 degrés jusqu'à la ligne médiane, le premier agencement ayant un premier volume total d'élément de fixation ; et
le deuxième agencement dudit au moins un élément de fixation (111B ; 211B ; 311B) intersectant le deuxième arc (154) ayant la deuxième distance radiale par rapport au point de référence (150) et s'étendant sur une plage angulaire de 90 degrés jusqu'à la ligne médiane, le deuxième agencement ayant un deuxième volume total d'élément de fixation,
dans lequel la deuxième distance radiale est plus grande que la première distance radiale.

6. Implant selon la revendication 2 ou 5, dans lequel le premier agencement comprend plusieurs éléments de fixation et le deuxième agencement comprend plusieurs éléments de fixation.

7. Implant selon la revendication 5, comprenant en outre une deuxième plaque d'extrémité (112) prévue avec le corps (102).

8. Implant selon la revendication 2 ou 7, dans lequel la première plaque d'extrémité (110) et la deuxième plaque d'extrémité (112) sont formées d'un seul tenant avec le corps (102).

9. Implant selon la revendication 2 ou 7, dans lequel le corps (102) et la première plaque d'extrémité (110) comprennent un matériau polymère.

10. Implant selon la revendication 1 ou 5, dans lequel le premier volume total d'élément de fixation est au moins deux fois plus grand que le deuxième volume total d'élément de fixation.

11. Implant selon la revendication 1 ou 5, dans lequel le premier volume total d'élément de fixation est entre 1 et 6 fois plus grand que le deuxième volume total d'élément de fixation.

12. Implant selon la revendication 5, dans lequel ledit au moins un élément de fixation (111A ; 211A ; 311A) du premier agencement a une première largeur et ledit au moins un élément de fixation (111B; 211B; 311B) du deuxième agencement a une deuxième largeur, et la première largeur est au moins deux fois plus grande que la deuxième largeur.

13. Implant selon la revendication 1, dans lequel la première plaque d'extrémité (110) comprend en outre :
le point de référence (150) défini au niveau du bord postérieur (118) de la première plaque d'extrémité (110) ;
le premier agencement dudit au moins un élément de fixation (111A ; 211A; 311 A), le premier agencement ayant un premier volume total d'élément de fixation, et chaque élément de fixation du premier agencement étant approximativement situé à une première distance minimale par rapport au point de référence (150) ; et
le deuxième agencement dudit au moins un élément de fixation (111B ; 211B; 311B), le deuxième agencement ayant un deuxième volume total d'élément de fixation, et chaque élément de fixation du deuxième agencement étant approximativement situé à une deuxième distance minimale par rapport au point de référence (150),
dans lequel la première distance minimale est inférieure à la deuxième distance minimale.
